# EUROPEAN PATENT SPECIFICATION

(11) **EP 3 630 280 B1**
(45) Date of publication and mention of the grant of the patent: **31.03.2021**
(21) Application number: 18725160.8
(22) Date of filing: 11.05.2018
(51) Int. Cl.: A61N 5/06

(54) **DEVICE COMBINING LASER TECHNOLOGY WITH OZONE FOR TREATING FUNGAL INFECTIONS**
VORRICHTUNG ZUR KOMBINATION VON LASERTECHNIK MIT OZON ZUR BEHANDLUNG VON PILZINFEKTIONEN
DISPOSITIF COMBINANT LA TECHNOLOGIE LASER À L'OZONE POUR LE TRAITEMENT DES INFECTIONS FONGIQUES

(30) Priority: 31.05.2017 ES 201730753
(43) Date of publication of application: 08.04.2020
(73) Proprietor: Termosalud, S.L., 33213 Gijon (ES)
(72) Inventor: DEL VALLE FERNANDEZ, Ivan, 33211 Gijon (ES)
(74) Representative: Clarke Modet & Co.
(86) International application number: PCT/EP2018/062220
(87) International publication number: WO 2018/219621

(56) References cited:
- WO-A1-2009/132503
- GB-A- 2 491 314
- KR-A- 20070 039 212
- US-A1- 2003 153 962
- US-A1- 2008 255 498

## Description

### Field of the Invention

The present invention relates to the technical field of medical devices used to treat fungi, and specifically to laser technology combined with ozone used to treat fungal infections.

### Background of the Invention

Mycotic or fungal infections are caused by fungi that infect the skin. They are frequent, contagious diseases for which there is currently no universal treatment that is quick, painless, effective and without side effects.

Fungal infections on toenails and fingernails (onychomycosis) are found all over the world. The prevalence of this disease has increased considerably over the last few decades, reaching figures of up to 10% of the world's population according to several authors. That is, 800 million people are affected worldwide by fungal nail infections. It is precisely in highly developed industrialised countries where this chronic pathological profile, which does not tend to self-heal, has even greater importance.

Various treatments for these infections are known in the state of the art, including topical or oral antifungal medication, laser radiation and surgery. These treatments however, have major shortcomings, a high recurrence rate, and significant costs and risks, making onychomycosis a disease which is difficult to cure.

In order to improve the treatment, new and enhanced ways of treating such infections are being researched, particularly within the field of laser radiation in various forms, and in combination with other technologies.

One example is patent application WO2006076506 which describes treating diseased nails using radiation and/or another form of energy to substantially deactivate the source of the disease. The treatment can be repeated and can be followed by applying a topical cream or ointment to the diseased nail, the cuticle, and/or the surrounding tissue, or by administering medication (e.g. oral or intravenous) to prevent recurrence of the organism or to eliminate the unwanted organism.

Another example is patent application WO2011116134 which describes fungal infection therapy with low level laser by means of a portable laser device which can be used to treat infected hands or feet. The treatment may be repeated, and topical anti-fungal medication may be applied to aid in the effectiveness of the treatment.

However, it can be observed how all the laser treatments for this type of infection require the subsequent application of medication, reflecting the low efficiency of laser alone. In addition, the elimination of onychomycosis through direct application of laser is not successful due to the difficulties of laser energy penetrating deeper levels of the nail without causing collateral damage.

KR 2007 0039212 A teaches a device combining ozone therapy with laser irradiation in a visible to near infrared range.

### Description of the Invention

The present invention resolves the existing problems in the state of the art by means of a device to apply laser technology at different wavelengths in combination with ozone in a gaseous state.

To do so it uses a device as defined in claim 1, comprising a plurality of laser emitters arranged in an electronic board, the light from which is radiated on a treatment chamber where a special sealed bag containing the affected limb is introduced, and which is pumped with a pre-established concentration of ozone by means of an ozone generating system.

In one aspect of the invention the laser system is located in the upper part of the treatment chamber such that the average energy density is the same at all points.

In another aspect of the invention, the laser system incorporates the device comprising laser emitters which emit at two wavelength ranges. In addition, some emitters emit wavelengths within the range of 350nm to 480nm, and the remaining laser emitters emit wavelengths in the range of 590nm to 750nm. These emitters are combined on a printed circuit board in such a way as to generate light radiation with the presence of both wavelengths, the energy density of which is applied uniformly on the affected area inserted in the treatment chamber.

These laser emitters are highly efficient, and have long service lives, reduced size, and low power consumption.

In another aspect of the invention, the ozone generating system comprises a component fixing tray, an oxygen inlet connector through which medicinal oxygen from a cylinder is introduced to the device, and a device ozone outlet female connector.

In another aspect of the invention, this component fixing tray comprises:
- an ozone generator, through which high voltage produces ozone from oxygen,
- solenoid valves to automate the system to carry out initial emptying, filling, treatment, and drain-disposal cycles,
- a vacuum pump used to initially empty the bag, and to subsequently drain and dispose of the ozone,
- an ozone destructor which is a catalytic element to destroy ozone and return oxygen to the atmosphere after the treatment, and
- printed circuit boards to control said components.

In another aspect of the invention, the bag comprises a connector located on one side of the bag which has been designed in such a way that it guarantees the ozone is sealed inside the bag. This connector is joined to a connecting tube which has a bag ozone inlet male connector at its opposite end.

In another aspect of the invention, the bag has a textile fixing structure which ensures that the bag is fixed over the limb, thereby preventing ozone leaks, and ensuring the sealing efficiency is not lost. In addition, the bag is removable and disposable (one per treatment) for hygiene reasons, and due to deterioration or alteration caused by the continuous contact with the ozone.

In another aspect of the invention, the device has a screen controlled by a microprocessor in order to set parameters of laser emission time, ozone concentration, and the wavelength of the laser emitters, so that it can be adapted to the needs or nature of the type of fungus to treat. This screen can be used to control and exchange information with the user, program sessions, control and adjust parameters, etc.

In another aspect of the invention, the device has an emergency button to stop any ozone generation or laser emission in the event of an electrical failure and/or ozone leak, thereby making the device and its use safer.

In an aspect of the disclosure, the device is used in an effective method to treat fungal infections.

The device demonstrates that the combination of ozone gas and laser light at certain wavelengths can eliminate infection causing fungal pathogens in a non-invasive and painless way without side effects, it therefore being suitable to be used to treat fungal infections.

The combined use of both technologies enhances the oxidative stress generated directly by an atmosphere enriched with ozone, allowing the concentrations of ozone used in the treatment and the ozone exposure time to be decreased. To do so, the device allows the treatment parameters to be set (ozone concentration, wavelengths, treatment duration, sequence, etc.) for each type of fungus.

Consequently, both the ozone exposure time and the concentration required to treat the tissues are minimised. In this way, the fungicidal effect of the treatment is enhanced, and its effectiveness maximised, thereby achieving a positive result by combining both technologies.

Therefore, the device is able to optimise the results in comparison with current methods, and it can reduce the effective time to achieve a clinical cure in patients with nails affected by a first infection or recurring onychomycosis.

### Brief Description of the Drawings

For the purpose of aiding to better understand the invention, a set of drawings is attached in which one embodiment has been depicted with an illustrative and nonlimiting character.
Figure 1 shows a perspective view of the device without the bag, prior to use, in which the device ozone outlet female connector cover is closed.
Figure 2 shows a perspective view of the device without the bag, prior to use, in which the device ozone outlet female connector cover is open.
Figure 3 shows a perspective view of the back of the device wherein the medicinal oxygen inlet connector is located.
Figure 4 shows a perspective view of the device with the bag connected to the device ozone outlet for it to be used.
Figure 5 shows a vertical section of the device during use with the bag connected to the device ozone outlet and introduced into the treatment chamber with the laser system activated.
Figure 6 shows a front view of the electronic board and the laser emitters, showing the arrangement of each of the types of device laser emitters. It also illustrates the emission rays of each type of laser emitter.
Figure 7 shows a perspective view of the bag with a closing element and the connecting tube attached.

These figures reference the following set of elements:
1. Device
2. Treatment chamber
3. Component fixing tray
4. Bag
5. Bag textile fixing structure
6. Sealing connector
7. Bag connecting tube
8. Bag ozone inlet male connector
9. Device ozone outlet female connector
10. Device ozone outlet female connector cover
11. Emergency button
12. Screen
13. Electronic board
14. Laser emitters
15. On/off button
16. Device oxygen inlet connector

### Detailed Description of the Invention

In view of the aforementioned drawings, and according to the adopted numbering, an example of a preferred embodiment of the invention can be observed, which comprises the parts and elements listed and described below.

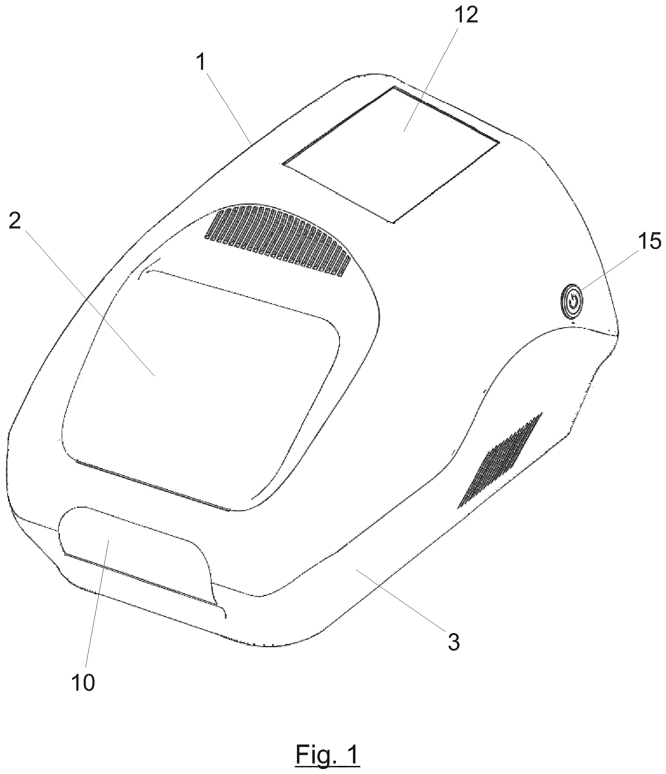

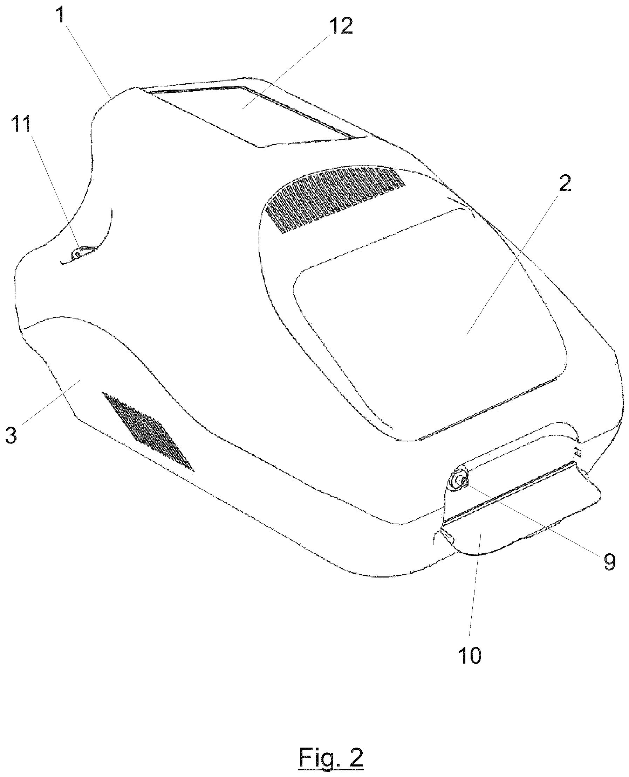

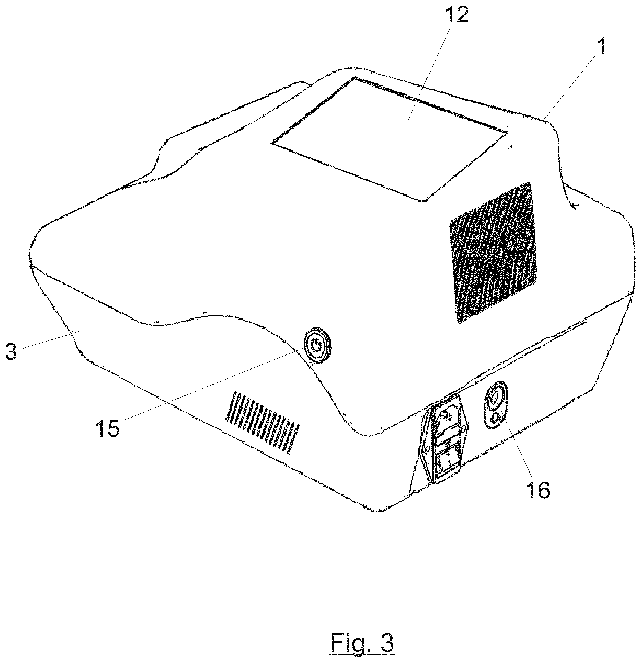

Figures 1 to 3 show the device (1), without the bag (4), depicting the treatment chamber (2) and the main elements of the ozone generating system: the component fixing tray (3), the device oxygen inlet connector (16) and the device ozone outlet female connector (9).

The upper part of the device (1) housing has a screen (12) controlled by a microprocessor to set the parameters of treatment duration, ozone concentration, laser emitter wavelength and application sequence, based on the user's requirements.

At the back of the device (1) there is an oxygen inlet connector (16), while at the front of the device (1) there is an ozone outlet female connector (9).

The component fixing tray (3) houses an ozone generator, solenoid valves, a vacuum pump, an ozone destructor, power sources, and printed circuit boards to control said components.

When the bag ozone inlet male connector (8) and the device ozone outlet female connector (9) are not connected, the cover (10) is kept closed to protect the device ozone outlet female connector (9) from potential damage.

The sides of the device incorporate an emergency button (11) on one side to stop any ozone generation or laser emission in the event of an electrical failure and/or ozone leak, and an on/off button (15) for the device (1) on the other side.

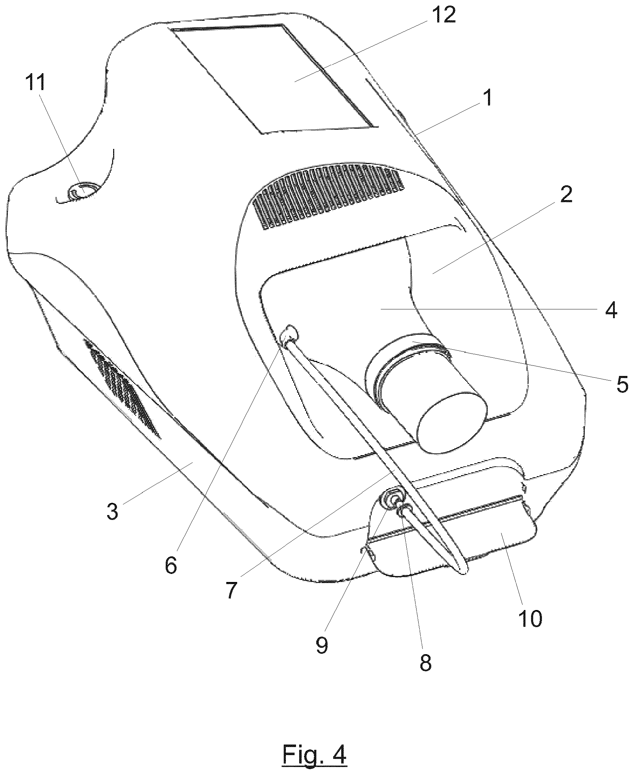

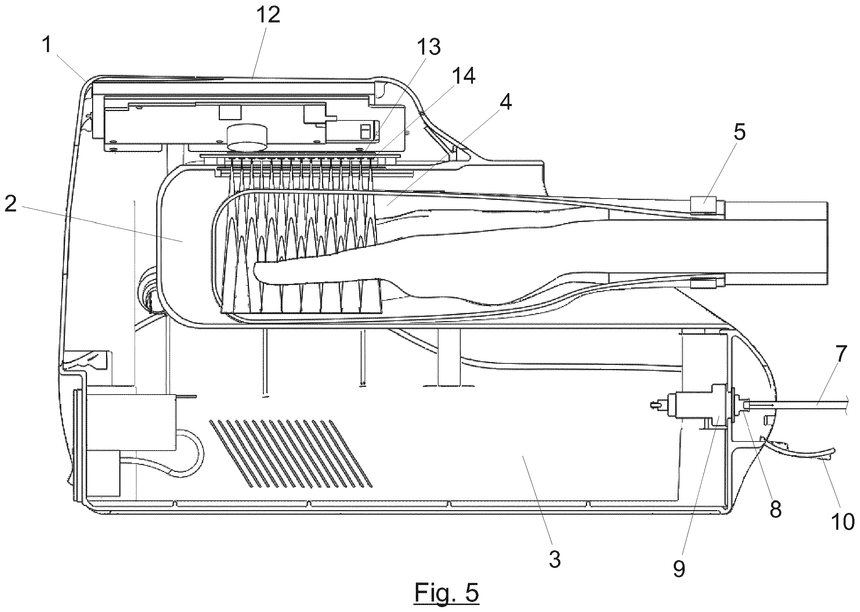

As can be observed in figures 4 and 5, the device (1) has a laser emission system formed by a plurality of laser emitters (14) arranged in an electronic board (13) located above the treatment chamber (2), a component fixing tray (3), and a bag (4) connected to the device (1) by means of connectors (8,9) and a tube (7). Said tube (7) is connected to the bag (4) by means of a sealing connector (6) which is heat sealed to the bag (4).

The bag (4) has a textile fixing structure (5) which allows the bag to be fixed to the user, thereby preventing ozone leaks.

As can be observed in figure 5, the laser emitters (14) are fixed, arranged in a grid on the electronic board (13) located in the upper part of the treatment chamber (2) in order to radiate the user's entire hand or foot, such that the average energy density is the same at all points of the treatment chamber (2).

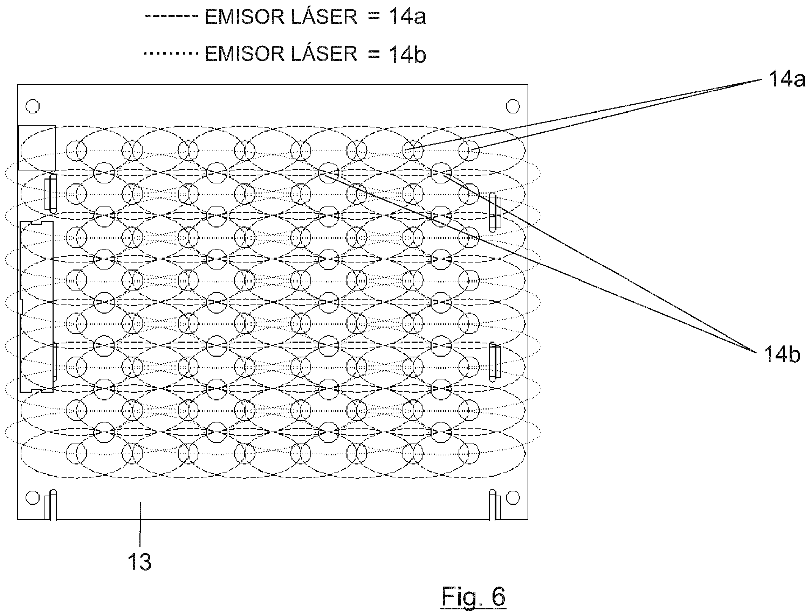

In more detail, figure 6 illustrates the preferred arrangement of the emitters in the electronic board (13). It can be observed that there are two types of emitters:
- Laser emitters (14a) with an emission range from 350nm to 480nm, and
- laser emitters (14b) with an emission range from 590nm to 750nm,
which are combined together to obtain homogeneous dispersion throughout the treatment chamber (2).

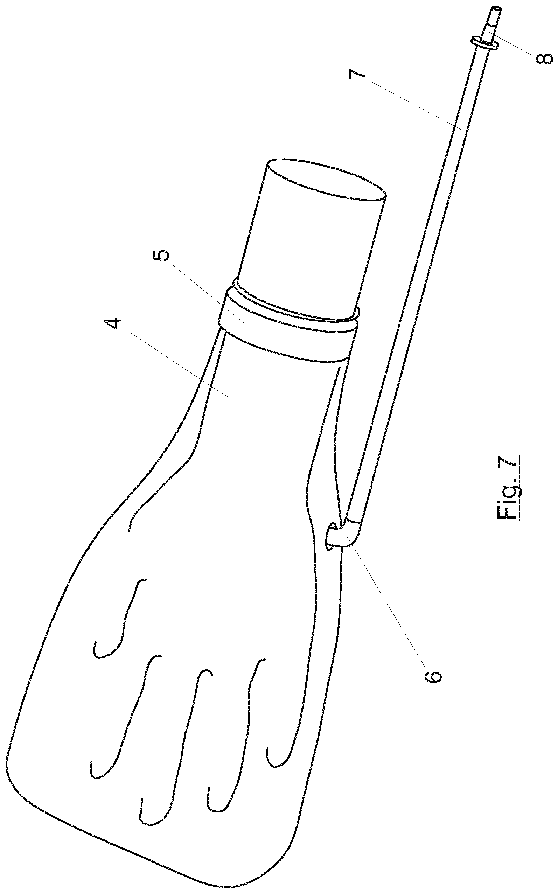

Figure 7 illustrates the bag (4) where the ozone is concentrated, which has a textile fixing structure (5) to prevent leaks, and a connecting tube (7) between the bag ozone inlet male connector (8) and a sealing connector (6) for the bag where the ozone is introduced.

After clearly describing the invention, it should be noted that the aforementioned particular embodiments are susceptible to changes in some details.

## Claims

1. A device (1) combining laser technology with ozone to treat fungal infections, comprising:
- a laser system comprising a plurality of laser emitters (14) arranged in an electronic board (13),
- an ozone generating system,
- a bag (4) wherein the ozone is concentrated;
wherein the bag (4) comprises a textile fixing structure (5) and a connector (6) located on one side of the bag which is joined to a connecting tube (7) having a bag ozone inlet male connector (8) at its opposite end; and
wherein the laser system comprises laser emitters (14a) which emit at wavelengths from 350nm to 480nm, and laser emitters (14b) which emit at wavelengths from 590nm to 750nm.

2. The device (1) according to claim 1, comprising a treatment chamber (2), wherein the laser system is located in the upper part of said treatment chamber (2).

3. The device (1) according to claims 1 to 2, **characterised in that** the ozone generating system comprises a component fixing tray (3), a device oxygen inlet connector (16), and a device ozone outlet female connector (9).

4. The device (1) according to claims 1 to 3, **characterised in that** the component fixing tray (3) comprises an ozone generator, solenoid valves, a vacuum pump, an ozone destructor, power sources, and printed circuit boards to control said components.

5. The device (1) according to claims 1 to 4, **characterised in that** it comprises a screen (12) controlled by a microprocessor.

6. The device (1) according to claims 1 to 5, **characterised in that** it comprises an emergency button (11).

## Patentansprüche

1. Vorrichtung (1) zur Kombination von Lasertechnik mit Ozon zur Behandlung von Pilzinfektionen, Folgendes umfassend:
- ein Lasersystem, das eine Vielzahl von Laseremittern (14) umfasst, die in einer Elektronikplatine (13) angeordnet sind,
- ein Ozonerzeugungssystem,
- einen Beutel (4), in dem das Ozon konzentriert wird;
wobei der Beutel (4) eine textile Befestigungsstruktur (5) und einen Anschluss (6) umfasst, der sich auf einer Seite des Beutels befindet und mit einem Verbindungsrohr (7) verbunden ist, das an seinem entgegengesetzten Ende einen Beutel-Ozoneinlassstecker (8) aufweist; und
wobei das Lasersystem Laseremitter (14a) umfasst, die bei Wellenlängen von 350 nm bis 480 nm emittieren, und Laseremitter (14b), die bei Wellenlängen von 590 nm bis 750 nm emittieren.

2. Vorrichtung (1) gemäß Anspruch 1, die eine Behandlungskammer (2) umfasst, wobei das Lasersystem im oberen Teil der Behandlungskammer (2) angeordnet ist.

3. Vorrichtung (1) gemäß Anspruch 1 bis 2, **dadurch gekennzeichnet, dass** das Ozonerzeugungssystem eine Komponentenbefestigungsschale (3), einen Vorrichtungs-Sauerstoffeinlassanschluss (16) und eine Vorrichtungs-Ozonauslassbuchse (9) umfasst.

4. Vorrichtung (1) gemäß Anspruch 1 bis 3, **dadurch gekennzeichnet, dass** die Komponentenbefestigungsschale (3) einen Ozonerzeuger, Magnetventile, eine Vakuumpumpe, einen Ozonzerstörer, Stromquellen und Leiterplatten zur Steuerung der Komponenten umfasst.

5. Vorrichtung (1) gemäß einem der Ansprüche 1 bis 4, **dadurch gekennzeichnet, dass** sie ein Display (12) umfasst, das von einem Mikroprozessor gesteuert wird.

6. Vorrichtung (1) gemäß Anspruch 1 bis 5, **dadurch gekennzeichnet, dass** sie einen Notdruckknopf (11) umfasst.

## Revendications

1. Un dispositif (1) combinant la technologie laser et l'ozone pour traiter les infections fongiques, comprenant :
- un système laser comprenant une pluralité d'émetteurs laser (14) disposés dans une carte électronique (13),
- un système générateur d'ozone,
- un sac (4) dans lequel l'ozone est concentré ;
dans lequel le sac (4) comprend une structure de fixation textile (5) et un connecteur (6) situé sur un côté du sac qui est relié à un tube de connexion (7) ayant un connecteur mâle (8) d'entrée d'ozone du sac à son extrémité opposée ; et
dans lequel le système laser comprend des émetteurs laser (14a) qui émettent à des longueurs d'onde de 350 nm à 480 nm, et des émetteurs laser (14b) qui émettent à des longueurs d'onde de 590 nm à 750 nm.

2. Le dispositif (1) selon la revendication 1, comprenant une chambre de traitement (2), dans lequel le système laser est situé dans la partie supérieure de ladite chambre de traitement (2).

3. Le dispositif (1) selon les revendications 1 à 2, **caractérisé en ce que** le système générateur d'ozone comprend un plateau de fixation des composants (3), un connecteur d'entrée d'oxygène du dispositif (16) et un connecteur femelle de sortie d'ozone du dispositif (9).

4. Le dispositif (1) selon les revendications 1 à 3, **caractérisé en ce que** le plateau de fixation des composants (3) comprend un générateur d'ozone, des électrovannes, une pompe à vide, un destructeur d'ozone, des sources d'énergie et des cartes de circuits imprimés pour contrôler lesdits composants.

5. Le dispositif (1) selon les revendications 1 à 4, **caractérisé en ce qu'**il comprend un écran (12) commandé par un microprocesseur.

6. L'appareil (1) selon les revendications 1 à 5, **caractérisé en ce qu'**il comprend un bouton d'urgence (11).
